# EUROPEAN PATENT APPLICATION

(11) **EP 4 813 376 A1**
(43) Date of publication of application: **30.09.2026**
(21) Application number: 24894668.3
(22) Date of filing: 21.11.2024
(51) Int. Cl.: A61K 31/00, A61P 35/00

(54) **PHARMACEUTICAL COMPOSITION FOR PREVENTION OR TREATMENT OF BREAST CANCER**

(30) Priority: 21.11.2023 KR 20230162398; 20.11.2024 KR 20240166743
(71) Applicant: Astrion Inc., Seoul 02842 (KR)
(72) Inventor: JEONG, Geuk-Rae, Seoul 02842 (KR); PARK, Hee Jo, Seoul 02842 (KR)
(74) Representative: Dehns
(86) International application number: PCT/KR2024/018570
(87) International publication number: WO 2025/110777

(57) **Abstract**

The present invention relates to a use of a novel compound for the prevention, alleviation, or treatment of breast cancer, the novel compound of the present invention not only being capable of notably inhibiting the growth, proliferation, migration, tumorigenicity, and metastasis of breast cancer cells, but also being capable of simultaneously inhibiting ANO1 and EGFR in breast cancer cells, thereby being capable of exhibiting a stronger anticancer effect through the dual inhibition of the two proteins. In addition, the compound, when used in combination with an anticancer agent, may further enhance the efficacy of the anticancer agent, and may inhibit the resistance of the cancer. Thus, the compound itself may be used as a dual-target anticancer agent against ANO1 and EGFR, and may also be utilized as a combined preparation of an anticancer agent, and thus is expected to be utilized in various ways in the field of breast cancer prevention and treatment.

## Description

### [Technical Field]

The present invention relates to a use of a novel compound for the prevention, amelioration, or treatment of breast cancer.

This application claims priority to and the benefit of Korean Patent Application No. 10-2023-0162398, filed on November 21, 2023, and Korean Patent Application No. 10-2024-0166743, filed on November 20, 2024, the disclosure of which is incorporated herein by reference in its entirety.

### [Background Art]

Breast cancer refers to cancer that originates in the lactiferous ducts and lobules of the breast. Currently, various factors such as the administration of female hormones, family history, medical history, childbirth history, and dietary habits are being discussed as causes of breast cancer, but none have been clearly identified yet. The breast cancer incidence among modern women is rapidly increasing, and along with factors such as low birth rates, short breastfeeding periods, early menarche, and late menopause, the incidence is also increasing among women in their 20s and 30s.

Clinically, breast cancer is classified into three subgroups based on the presence or absence of the expression of major genes: 1) estrogen receptor (ER)-positive, which expresses the estrogen receptor; 2) Human epidermal growth factor receptor 2 (HER2)-positive, which expresses HER2; and 3) triple-negative breast cancer, which does not express any of the three genes: ER, progesterone receptor (PR), and HER2.

Epidermal growth factor receptor (EGFR) is a receptor for epidermal growth factor (EGF), which is a peptide that promotes the growth of breast cancer cells, and is present across the inner and outer layers of the cell membrane. EGFR promotes cell growth and DNA synthesis by activating EGF, transforming growth factor α, and tyrosine kinase. The presence of EGFR is observed not only in breast cancer but also in lung, gastric, and liver cancers. In particular, EGFR is known to play a crucial role in carcinogenesis and tumor progression in breast cancer. Regarding the prognosis of breast cancer, the expression of EGFR is closely associated with histologic grading and lymphatic invasion, and it is known that recurrence and mortality rates are higher in EGFR-positive breast cancer compared to EGFR-negative breast cancer.

Meanwhile, calcium-activated chloride channels (CaCCs) are widely expressed in various cells and tissues and serve to regulate physiological functions such as epithelial fluid secretion, smooth muscle contraction, sensory nerve signaling, and cell growth. After anoctamin-1/transmembrane member 16 (AANO1/TMEM16A) was identified as a CaCC in 2008, various studies have shown that anoctamin-1 (ANO1) is highly expressed in the bronchi, intestines, glandular epithelium, smooth muscles, intestinal pacemaker cells, sensory nerves, and various types of tumor cells such as head and neck cancer, breast cancer, prostate cancer, and kidney cancer, and that ANO1 possesses diverse physiological functions.

ANO1 is located on chromosome band 11q13 and is overexpressed in various cancer cells, such as head and neck cancer, breast cancer, and prostate cancer. Recent research has revealed that ANO1 is involved in cell growth, migration, cancerization, and cancer development. For example, in a mouse model, it was observed that inhibiting the expression of ANO1 in prostate cancer cells (PC3) suppressed cell growth, metastasis, invasion, and tumor development, and the ANO1 inhibitor T16Ainh-A01 suppressed the growth of interstitial cells of Cajal (ICC) and pancreatic cancer cells (CFPAC1) that express high levels of ANO1. Furthermore, it has been reported that inhibiting the expression of ANO1 in breast cancer cells suppresses cell growth, induces apoptosis, and inhibits tumor growth in a xenograft model. In addition, it has been found that inhibiting ANO1 exhibits an anti-cancer effect by inhibiting EGFR and calmodulin-dependent protein kinase 2 (CAMK2) signaling in head and neck cancer (head and neck squamous cell carcinoma (HNSCC)), esophageal squamous cell carcinoma (ESCC), and breast cancer cells. Therefore, recent research results suggest that pharmacological inhibition of ANO1 ion channel activity has potential as a treatment for head and neck cancer, esophageal cancer, gastrointestinal cancer, breast cancer, and prostate cancer that overexpress ANO1.

Thus, both ANO1 and EGFR proteins have the potential to be therapeutic targets for breast cancer. However, no substance capable of exhibiting excellent anti-cancer effects through the dual inhibition of ANO1 and EGFR has yet been discovered.

### [Disclosure]

### [Technical Problem]

The present inventors have completed the present invention by confirming that the novel compound of the present invention inhibits the growth, proliferation, migration, tumorigenicity, and metastasis of breast cancer cells, simultaneously inhibits ANO1, which is a calcium-dependent chloride channel, and EGFR, which is an epidermal growth factor receptor, thereby not only exhibiting an excellent anti-cancer effect, but also exhibiting a high synergistic effect when co-administered with an anti-cancer agent.

Therefore, an object of the present invention is to provide a pharmaceutical composition for prevention or treatment of breast cancer, comprising, as an active ingredient, a compound represented by Chemical Formula 1 below or a pharmaceutically acceptable salt thereof.

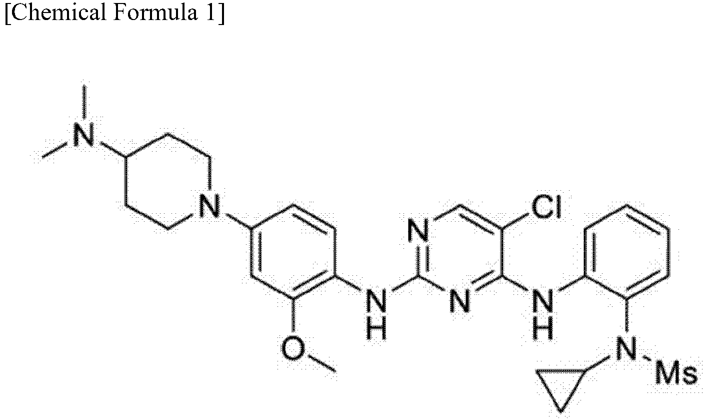

Another object of the present invention is to provide a pharmaceutical composition for prevention or treatment of breast cancer, comprising, as active ingredients, (i) the compound represented by Chemical Formula 1 or a pharmaceutically acceptable salt thereof; and (ii) one or more anti-cancer agents selected from the group consisting of chemical anti-cancer agents and targeted anti-cancer agents.

Yet another object of the present invention is to provide a pharmaceutical composition for enhancing an anti-cancer effect of an anti-cancer agent on breast cancer, comprising, as an active ingredient, the compound represented by Chemical Formula 1 or a pharmaceutically acceptable salt thereof.

However, technical problems to be solved in the present invention are not limited to the above-described problems, and other problems which are not described herein will be fully understood by those of ordinary skill in the art from the following descriptions.

### [Technical Solution]

In order to achieve the above object, the present invention provides a pharmaceutical composition for prevention or treatment of breast cancer, comprising, as an active ingredient, a compound represented by Chemical Formula 1 below or a pharmaceutically acceptable salt thereof.

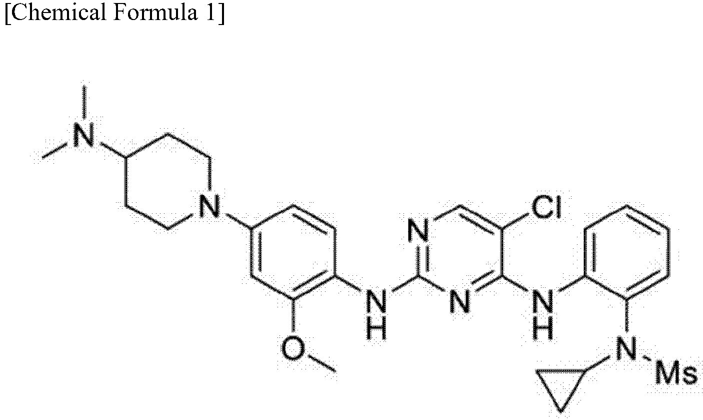

In one embodiment of the present invention, the breast cancer may be one or more selected from the group consisting of breast ductal carcinoma in situ, inflammatory breast cancer, triple-negative breast cancer, invasive ductal carcinoma, invasive lobular carcinoma, ductal carcinoma in situ (non-invasive), lobular carcinoma *in situ* (non-invasive), Paget's disease of the breast, invasive breast cancer, and metastatic breast cancer, but is not limited thereto.

In another embodiment of the present invention, the compound or a pharmaceutically acceptable salt thereof may simultaneously inhibit epidermal growth factor receptor (EGFR) and anoctamin-1 (ANO1), but is not limited thereto.

In yet another embodiment of the present invention, the compound or a pharmaceutically acceptable salt thereof may satisfy one or more properties selected from the group consisting of the following, but is not limited thereto:
(a) inhibiting the proliferation or growth of cancer cells;
(b) inhibiting the migration of cancer cells;
(c) inhibiting the tumorigenicity of cancer cells;
(d) inhibiting the metastasis of cancer; and
(e) inhibiting the resistance of cancer to anti-cancer agents.

In yet another embodiment of the present invention, the anti-cancer agent may be a chemical anti-cancer agent or a targeted anti-cancer agent, but is not limited thereto.

In yet another embodiment of the present invention, the chemical anti-cancer agent may be one or more selected from the group consisting of paclitaxel, docetaxel, dactinomycin, doxorubicin, daunorubicin, mitomycin, and bleomycin, but is not limited thereto.

In yet another embodiment of the present invention, the targeted anti-cancer agent may be one or more selected from the group consisting of a targeted anti-cancer agent for EGFR, a targeted anti-cancer agent for trophoblast cell-surface antigen 2 (TROP-2), and a targeted anti-cancer agent for poly(ADP-ribose) polymerase (PARP), but is not limited thereto.

In yet another embodiment of the present invention, the pharmaceutical composition may be administered in combination with a chemical anti-cancer agent or a targeted anti-cancer agent, but is not limited thereto.

In yet another embodiment of the present invention, the pharmaceutical composition may be administered simultaneously, separately, or sequentially with the anti-cancer agent, but is not limited thereto.

Additionally, the present invention provides a pharmaceutical composition for prevention or treatment of breast cancer, comprising, as active ingredients, (i) the compound represented by Chemical Formula 1 or a pharmaceutically acceptable salt thereof; and (ii) one or more anti-cancer agents selected from the group consisting of chemical anti-cancer agents and targeted anti-cancer agents.

In one embodiment of the present invention, the compound or a pharmaceutically acceptable salt thereof may inhibit the resistance of cancer cells to the anti-cancer agent, but is not limited thereto.

In another embodiment of the present invention, the composition may be in the form of a mixture mixing the compound or a pharmaceutically acceptable salt thereof; and the anti-cancer agent, but is not limited thereto.

In yet another embodiment of the present invention, the composition may be in a form in which the compound or a pharmaceutically acceptable salt thereof; and the anti-cancer agent are each formulated to be administered simultaneously, separately, or sequentially, but is not limited thereto.

Additionally, the present invention provides a pharmaceutical composition for enhancing an anti-cancer effect of an anti-cancer agent on breast cancer, comprising, as an active ingredient, the compound represented by Chemical Formula 1 or a pharmaceutically acceptable salt thereof.

In one embodiment of the present invention, the anti-cancer agent may be one or more selected from the group consisting of chemical anti-cancer agents and targeted anti-cancer agents, but is not limited thereto.

In another embodiment of the present invention, the chemical anti-cancer agent may be one or more selected from the group consisting of paclitaxel, docetaxel, dactinomycin, doxorubicin, daunorubicin, mitomycin, and bleomycin, but is not limited thereto.

In yet another embodiment of the present invention, the targeted anti-cancer agent may be one or more selected from the group consisting of a targeted anti-cancer agent for EGFR, a targeted anti-cancer agent for TROP-2, and a targeted anti-cancer agent for PARP, but is not limited thereto.

In yet another embodiment of the present invention, the composition may be administered simultaneously, separately, or sequentially with the anti-cancer agent, but is not limited thereto.

Additionally, the present invention provides a method of preventing, ameliorating, or treating breast cancer, or a method of enhancing an anti-cancer effect of an anti-cancer agent on breast cancer, comprising administering the compound represented by Chemical Formula 1 or a pharmaceutically acceptable salt thereof; or a composition comprising the same, in a pharmaceutically effective amount to a subject in need thereof.

Additionally, the present invention provides a use of the compound represented by Chemical Formula 1 or a pharmaceutically acceptable salt thereof; or a composition comprising the same for preventing, ameliorating, or treating breast cancer; or for enhancing an anti-cancer effect of an anti-cancer agent on breast cancer.

Additionally, the present invention provides a use of the compound represented by Chemical Formula 1 or a pharmaceutically acceptable salt thereof; or a composition comprising the same for preparing a preparation for preventing, ameliorating, or treating breast cancer; or for preparing a preparation for enhancing an anti-cancer effect of an anti-cancer agent on breast cancer.

Additionally, the present invention provides a method of preventing, ameliorating, or treating breast cancer, comprising administering (i) the compound represented by Chemical Formula 1 or a pharmaceutically acceptable salt thereof, and (ii) one or more anti-cancer agents selected from the group consisting of chemical anti-cancer agents and targeted anti-cancer agents; or a composition comprising the same, in a pharmaceutically effective amount to a subject in need thereof.

Additionally, the present invention provides a use of (i) the compound represented by Chemical Formula 1 or a pharmaceutically acceptable salt thereof, and (ii) one or more anti-cancer agents selected from the group consisting of chemical anti-cancer agents and targeted anti-cancer agents; or a composition comprising the same for preventing, ameliorating, or treating breast cancer.

Additionally, the present invention provides a use of (i) the compound represented by Chemical Formula 1 or a pharmaceutically acceptable salt thereof, and (ii) one or more anti-cancer agents selected from the group consisting of chemical anti-cancer agents and targeted anti-cancer agents; or a composition comprising the same for preparing a preparation for preventing, ameliorating, or treating breast cancer.

### [Advantageous Effects]

The novel compound of the present invention not only significantly inhibits the growth, proliferation, migration, tumorigenicity, and metastasis of breast cancer cells, but also may simultaneously inhibit ANO1 and EGFR in breast cancer cells, thereby exhibiting a stronger anti-cancer effect through the dual inhibition of the two proteins. Additionally, when used in combination with an anti-cancer agent and the like, the compound may further enhance the efficacy of the anti-cancer agent and may inhibit the resistance of cancer. Therefore, the compound itself may be used as a dual-target anti-cancer agent against ANO1 and EGFR, and may also be utilized as a combined preparation with an anti-cancer agent, and thus is expected to be utilized in various ways in the field of prevention and treatment of breast cancer.

### [Description of Drawings]

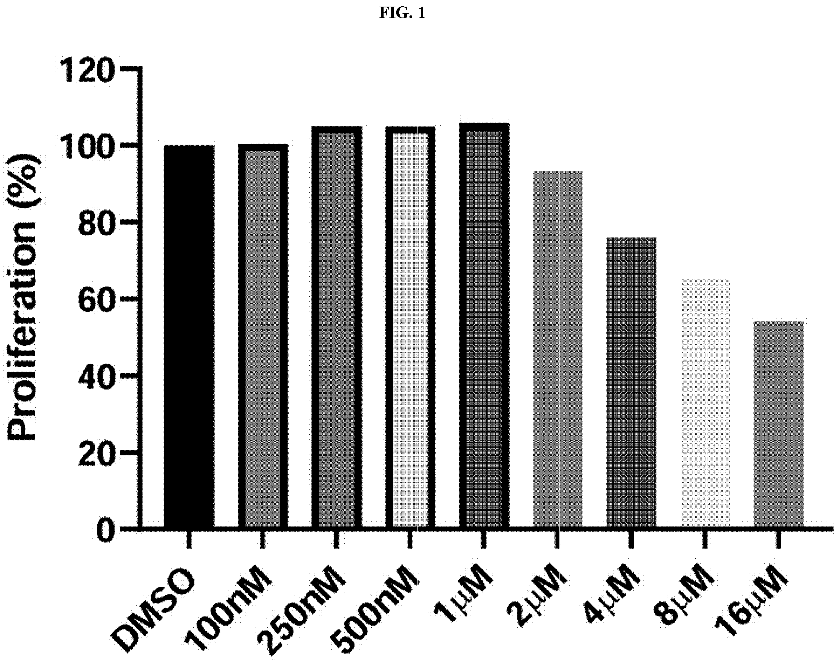

FIG. 1 shows the results of performing a CCK assay after treating human breast cancer cell line MDA-MB-231 cells with compound AON-MG23 of the present invention at various concentrations in order to confirm the proliferation inhibitory effect of compound AON-MG23 of the present invention on human breast cancer cells.

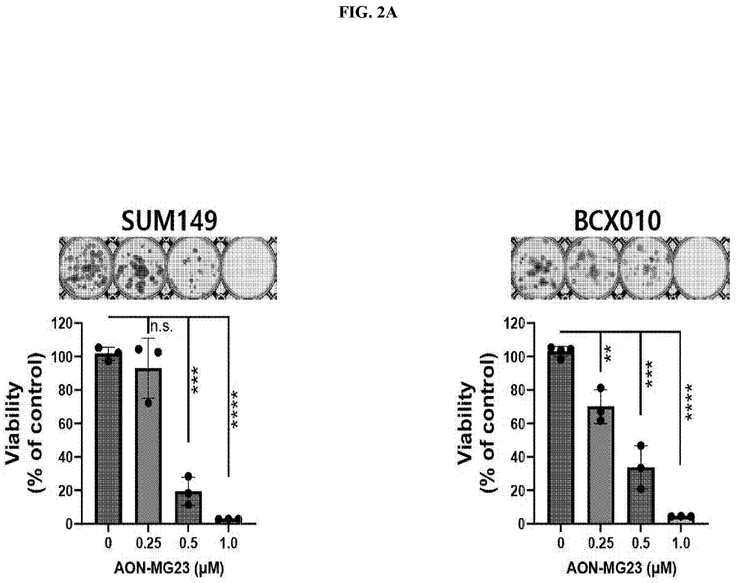

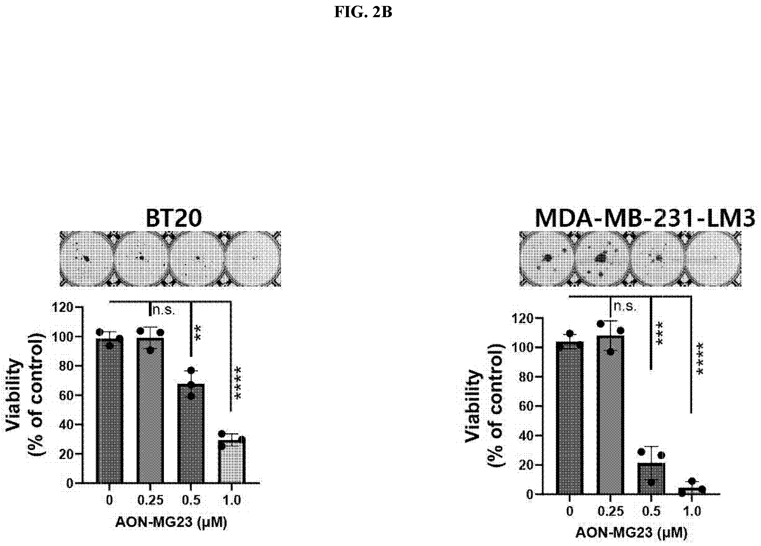

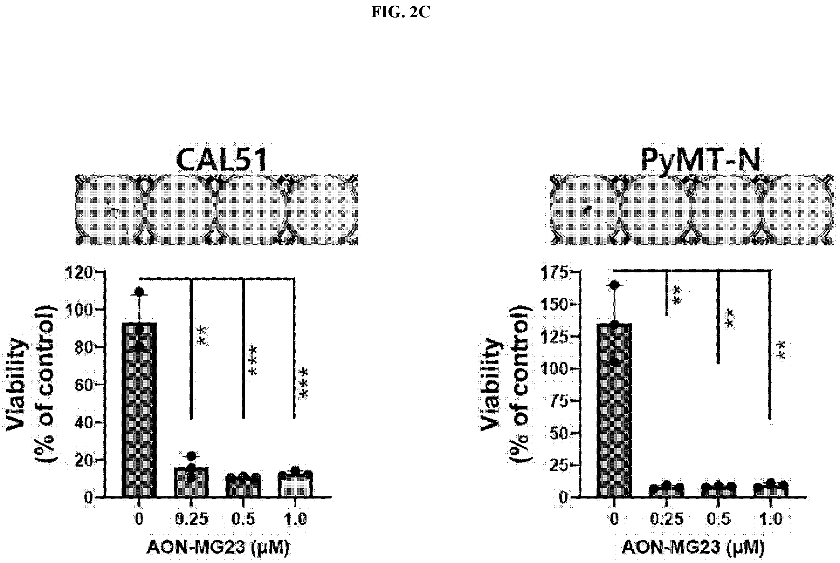

FIGS. 2A to 2C show the results of confirming cell colony-forming ability after treating six breast cancer cell lines (SUM149, BCX010, BT20, MDA-MB-231-LM3, CAL51, and PyMT-N) with compound AON-MG23 of the present invention at various concentrations in order to confirm the proliferation inhibitory effect of compound AON-MG23 of the present invention on human breast cancer cells.

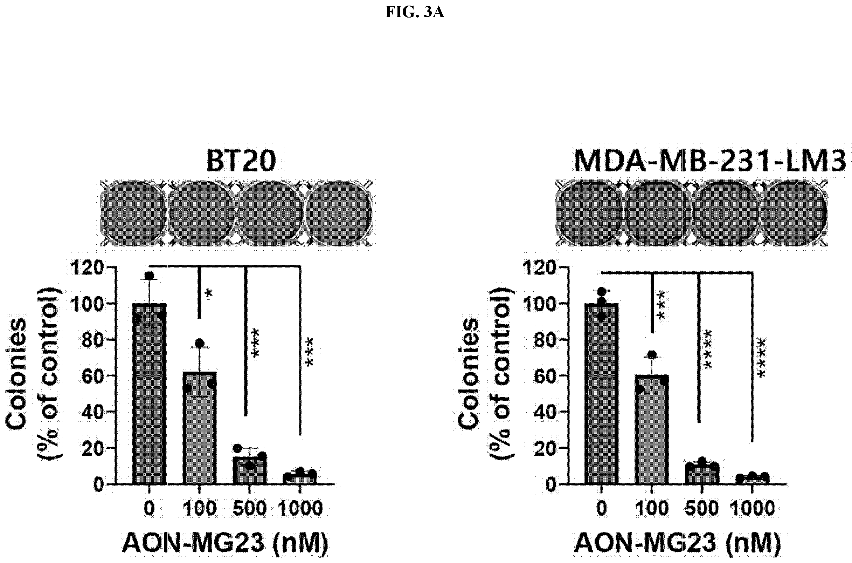

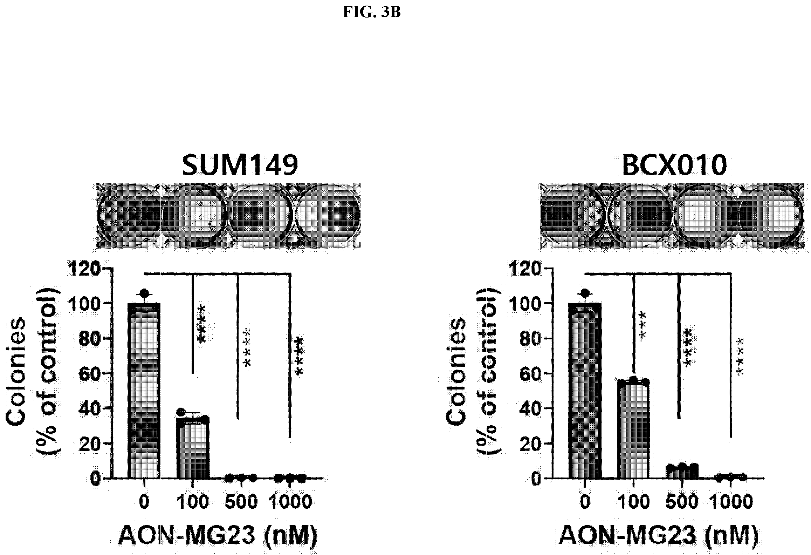

FIGS. 3A and 3B show the results of performing an anchorage-independent soft agar assay after treating four breast cancer cell lines (SUM149, BCX010, BT20, and MDA-MB-231-LM3) with compound AON-MG23 of the present invention at various concentrations in order to confirm the tumorigenicity inhibitory effect of compound AON-MG23 of the present invention on human breast cancer cells.

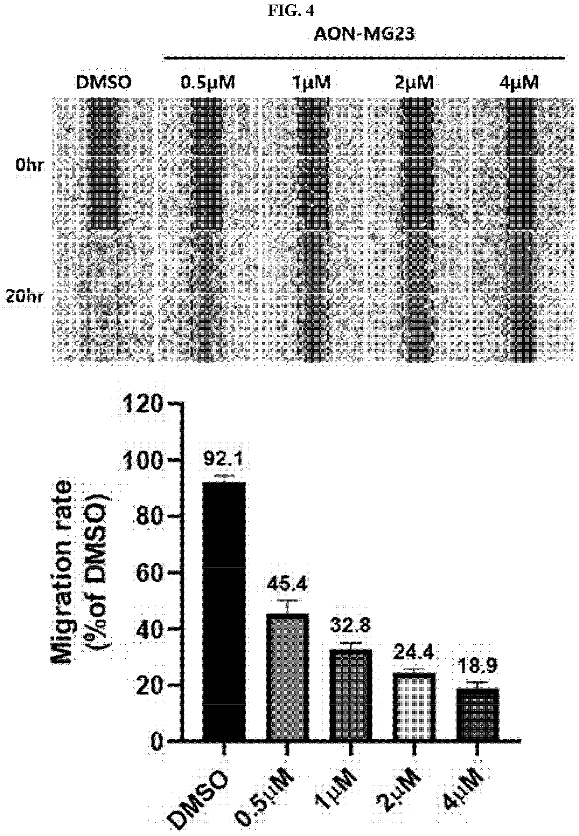

FIG. 4 shows the results of photographing and analyzing the degree of cell migration over time after treating human breast cancer cell line MDA-MB-231 cells with compound AON-MG23 of the present invention at various concentrations in order to confirm the cell migration inhibitory effect of compound AON-MG23 of the present invention on human breast cancer cells.

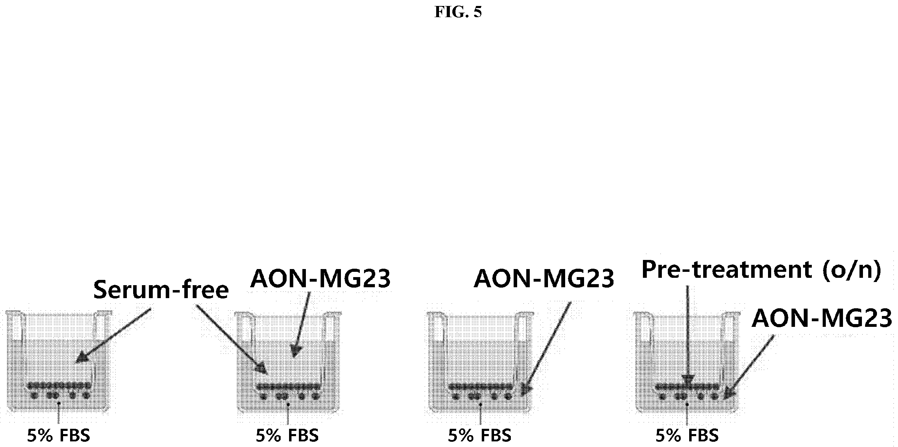

FIG. 5 shows a schematic diagram of an experiment for confirming the cell migration inhibitory effect of compound AON-MG23 of the present invention.

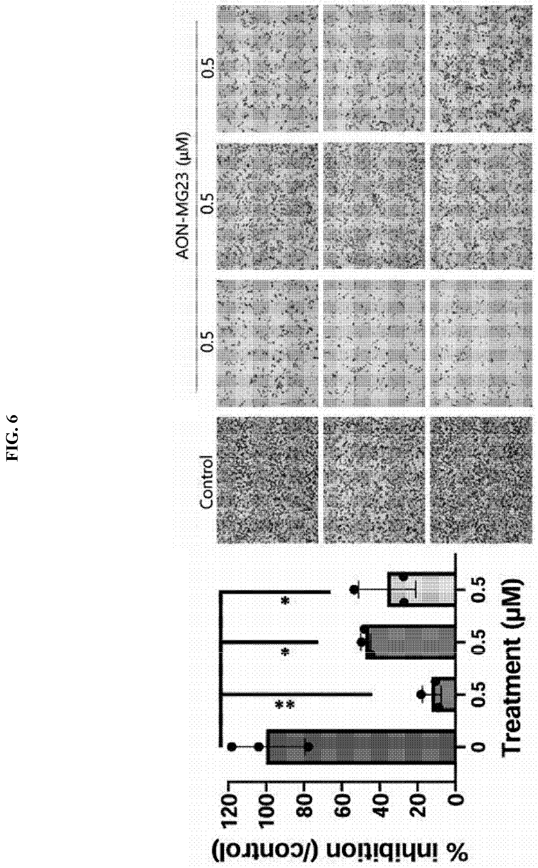

FIG. 6 shows the results of confirming migratory ability after pre-treating human breast cancer cell line MDA-MB-231-LM3 cells with compound AON-MG23 of the present invention at a single concentration (0.5 µM) in order to confirm the cell migration inhibitory effect of compound AON-MG23 of the present invention.

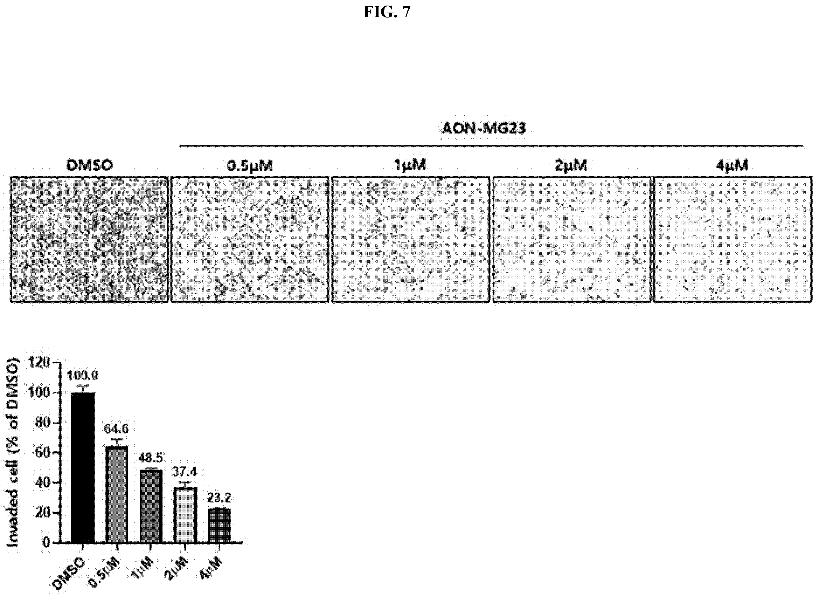

FIG. 7 shows the results of photographing and analyzing the degree of Matrigel invasion of cells after treating human breast cancer cell line MDA-MB-231 cells being cultured on Matrigel with compound AON-MG23 of the present invention at various concentrations in order to confirm the inhibitory effect of compound AON-MG23 of the present invention on breast cancer cell metastasis.

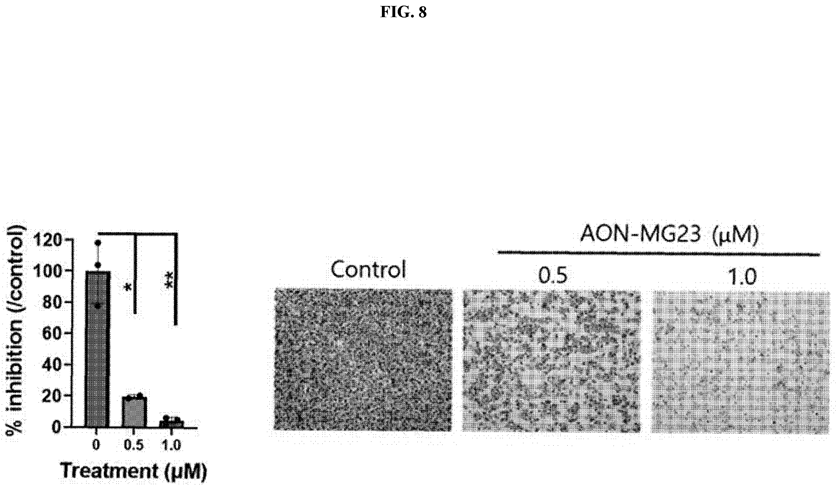

FIG. 8 shows the results obtained after treating human breast cancer cell line MDA-MB-231-LM3 cells with compound AON-MG23 of the present invention at different concentrations in order to confirm the inhibitory effect on cell metastasis in human breast cancer cells.

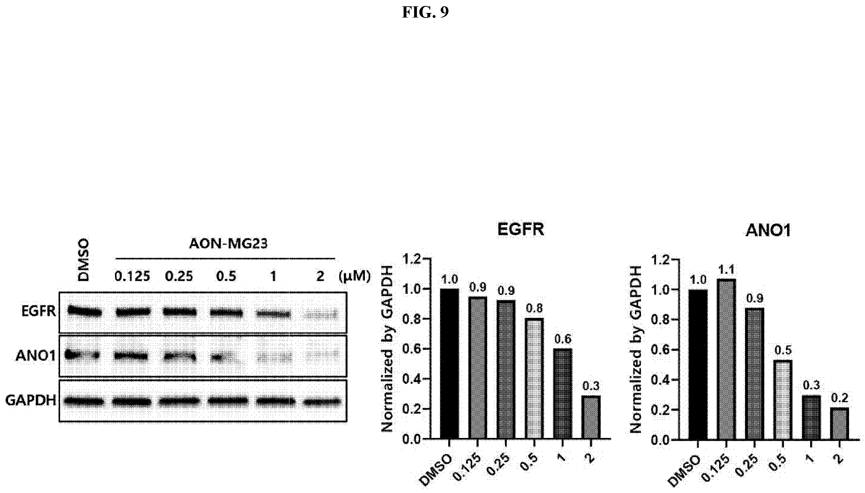

FIG. 9 shows the results of confirming the effects of compound AON-MG23 of the present invention on the expression of ANO1 protein and EGFR protein in human breast cancer cells.

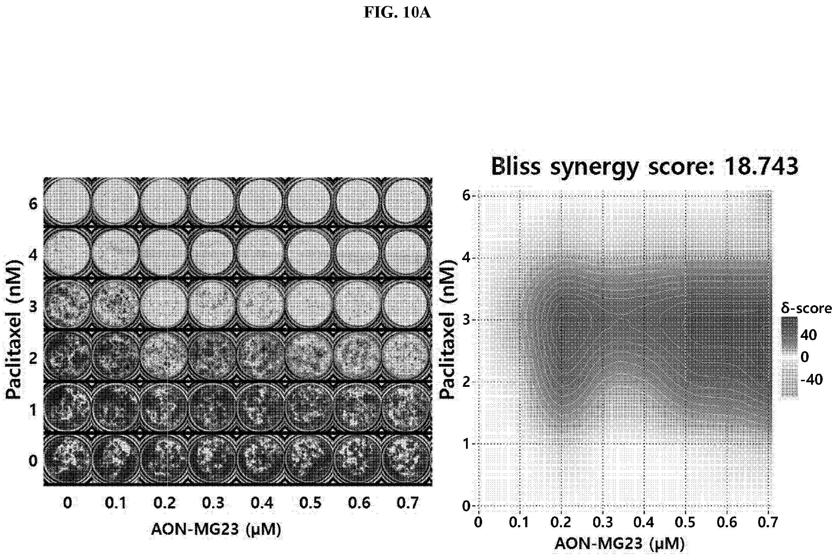

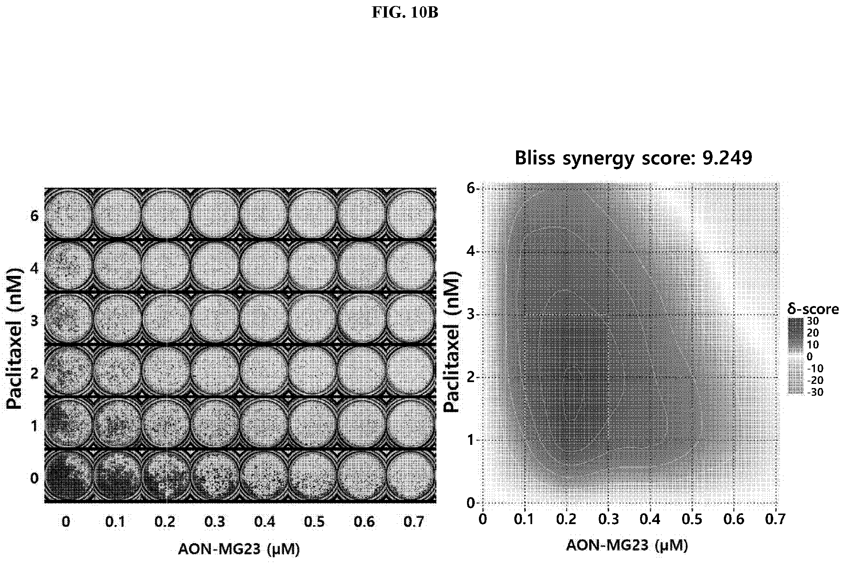

FIGS. 10A and 10B show the results of confirming the combination effect of compound AON-MG23 of the present invention with paclitaxel in human breast cancer cells. FIG. 10A shows human breast cancer cell line SUM149, and FIG. 10B shows human breast cancer cell line HCC1806.

### [Best Mods]

The present inventors have completed the present invention by confirming that the novel compound of the present invention inhibits the growth, proliferation, metastasis, tumorigenicity, and migration of breast cancer cells, simultaneously inhibits ANO1, which is a calcium-dependent chloride channel, and EGFR, which is an epidermal growth factor receptor, thereby not only exhibiting an excellent anti-cancer effect, but also exhibiting a high synergistic effect when co-administered with an anti-cancer agent.

Hereinafter, the present invention will be described in detail.

The present invention provides a pharmaceutical composition for prevention or treatment of breast cancer, comprising, as an active ingredient, a compound represented by Chemical Formula 1 below or a pharmaceutically acceptable salt thereof.

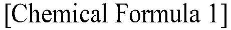

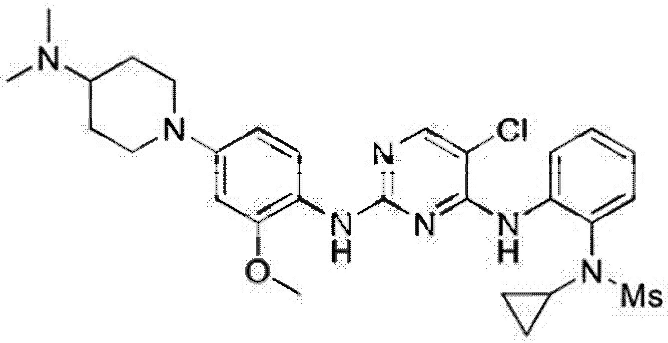

The compound represented by Chemical Formula 1 may be referred to as "N-(2-((5-chloro-2-((4-(4-(dimethylamino)piperidin-1-yl)-2-methoxyphenyl)amino)pyrimidin-4-yl)amino)phenyl)-N-cyclopropylmethanesulfonamide" or "AON-MG23," and in Chemical Formula 1, "Ms" means methylsulfonyl, that is, -SO₂(CH₃).

Hereinafter, unless otherwise stated, the term "compound of the present invention" or "compound represented by Chemical Formula 1" and the like are used as a concept including the compound represented by Chemical Formula 1 itself, salts thereof, isomers thereof, and the like.

In the present invention, the term "pharmaceutically acceptable salt" includes all salts derived from pharmaceutically acceptable inorganic acids, organic acids, or bases, and the like.

As used herein, the term "pharmaceutically acceptable" means a compound or composition that is suitable for use in contact with the tissues of a subject (for example, a human) without excessive toxicity, irritation, allergic response, or other problems or complications, having a reasonable benefit/risk ratio, and being within the scope of sound medical judgment.

Examples of suitable acids include hydrochloric acid, hydrobromic acid, sulfuric acid, nitric acid, perchloric acid, hydroiodic acid, fumaric acid, maleic acid, phosphoric acid, glycolic acid, lactic acid, salicylic acid, succinic acid, toluene-p-sulfonic acid, tartaric acid, (+)-L-tartaric acid, di-L-tartaric acid, acetic acid, trichloroacetic acid or trifluoroacetic acid, 2,2-dichloroacetic acid, acylated amino acids, adipic acid, alginic acid, ascorbic acid, L-aspartic acid, 4-acetamidobenzoic acid, (+)-camphoric acid, camphorsulfonic acid, (+)-(1S)-camphorsulfonic acid, capric acid, caproic acid, caprylic acid, cinnamic acid, cyclamic acid, dodecylsulfuric acid, ethane-1,2-disulfonic acid, ethanesulfonic acid, 2-hydroxyethanesulfonic acid, galactaric acid, gentisic acid, glucoheptanoic acid, D-gluconic acid, D-glucuronic acid, L-glutamic acid, a-oxo-glutaric acid, hippuric acid, (+)-L-lactic acid, (+-)-DL-lactic acid, lactobionic acid, (-)-L-malic acid, (+-)-DL-mandelic acid, citric acid, methanesulfonic acid, formic acid, benzoic acid, malonic acid, gluconic acid, naphthalene-2-sulfonic acid, naphthalene-1,5-disulfonic acid, benzenesulfonic acid, 1-hydroxy-2-naphthoic acid, nicotinic acid, oleic acid, orotic acid, oxalic acid, dioxalic acid, palmitic acid, palmic acid, L-pyroglutamic acid, salicylic acid, 4-amino-salicylic acid, sebacic acid, stearic acid, tannic acid, thiocyanic acid, camsylic acid, and undecylic acid. Acid addition salts may be prepared by conventional methods, for example, by dissolving the compound in an excess aqueous acid solution and precipitating the salt using a water-miscible organic solvent such as methanol, ethanol, acetone, or acetonitrile. Additionally, the acid addition salts may be prepared by heating an equimolar amount of the compound and the acid or alcohol in water and then evaporating the mixture to dryness, or by suction-filtering the precipitated salt.

The scope of the compound of the present invention may include not only pharmaceutically acceptable salts, but also all isomers, hydrates, and solvates that may be prepared by conventional methods.

In the present invention, the term "isomer" refers to a compound having the same molecular formula but differing in the connection manner of constituent atoms within the molecule or in the spatial arrangement thereof. The isomer includes, for example, a structural isomer and a stereoisomer. The stereoisomer may be a diastereomer or an enantiomer. The enantiomer refers to an isomer that does not overlap with its mirror image, like the relationship between the left hand and the right hand, and is also referred to as an optical isomer. The enantiomer is classified into R (Rectus: clockwise) and S (Sinister: counterclockwise) when four substituents on a chiral center carbon are different from each other. The diastereomer refers to a stereoisomer that is not in a mirror-image relationship, and may be divided into cis-trans isomers caused by differences in the spatial arrangement of atoms.

The compound of the present invention is characterized by exhibiting an excellent anti-cancer effect by simultaneously inhibiting the expression or activity of EGFR and ANO1. The prevention and/or treatment effect on the breast cancer includes not only an effect of inhibiting the growth of breast cancer cells, but also an effect of inhibiting progression or aggravation of breast cancer due to migration, invasion, metastasis, tumorigenicity, and the like.

In the present invention, "EGFR (epidermal growth factor receptor)" is a transmembrane glycoprotein that is a member of the protein kinase superfamily, and is a receptor for proteins of the epidermal growth factor family. EGFR is a major regulator of cell growth, and when epidermal growth factor (EGF), which is a ligand, binds to EGFR, the receptor causes dimerization and tyrosine autophosphorylation, thereby inducing cell proliferation. Overexpression (amplification) or hyperactivation of EGFR is frequently observed in various cancers such as anal cancer, breast cancer (particularly, triple-negative breast cancer), and glioblastoma, and somatic mutations associated with EGFR cause continuous activation of EGFR, thereby inducing uncontrolled cell division. In particular, EGFR gene rearrangement and EGFR gene amplification are patterns observed in various cancers.

In the present invention, "Anoctamin 1 (ANO1)" is a calcium-activated chloride ion channel (Cl- channel), which is also referred to as "transmembrane protein 16A (TMEM16A)." ANO1 is expressed in various normal cells including epithelial cells, smooth muscle cells, vascular endothelial cells, and neurons, and performs various physiological roles by regulating body fluid secretion, muscle contraction, nociception, and the like. However, ANO1 is also known to be involved in cancerization of cells and cancer development. In fact, overexpression of ANO1 promotes signaling pathways that stimulate proliferation and migration in cancer cells, and inhibition of ANO1 has been reported to inhibit migration and growth of cancer cells. High expression of ANO1 appears to be associated with high expression of EGFR and STAT3, and inhibition of ANO1 is also known to improve the response of head and neck squamous cell carcinoma to EGFR/HER2-targeted therapy. Therefore, ANO1 has attracted attention as a target for cancer treatment, but an ANO1 inhibitor that actually exhibits an excellent anti-cancer effect has not yet been discovered. The compound of the present invention effectively inhibits the expression of ANO1 in breast cancer cells, and has been confirmed to inhibit migration, proliferation, metastasis, and the like of breast cancer cells, and thus may be used for prevention, treatment, and inhibition of metastasis of breast cancer. Furthermore, previous studies on improvement of the effect of EGFR/HER2-targeted therapy by ANO1 inhibition suggest that the compound according to the present invention may increase responsiveness of cancer cells to EGFR/HER-targeted therapy through the ANO1 inhibitory effect and may inhibit development of resistance.

In particular, the compound of the present invention simultaneously inhibits the expression of EGFR together with ANO1 in breast cancer cells, and thus may exhibit a stronger anti-cancer effect through simultaneous inhibition of ANO1 and EGFR. The association between ANO1 and EGFR is well known, and upregulation of ANO1 is associated with the signaling pathway of EGFR and has been reported to have a significant effect on remodeling of the phosphorylated proteome after epidermal growth factor (EGF) stimulation. Additionally, in cancer cells, ANO1 may form a functional complex with EGFR to jointly regulate cell proliferation. Therefore, when EGFR and ANO1 are simultaneously inhibited by using the compound of the present invention, the growth of breast cancer may be more effectively inhibited.

Meanwhile, ANO1 is known to be overexpressed in cancer cells and contribute to migration of cancer cells and tumor metastasis, and signaling by EGFR is also known to generate a tumor microenvironment that aids tumor metastasis. Therefore, the compound according to the present invention may effectively inhibit metastasis of breast cancer through simultaneous inhibition of ANO1 and EGFR. The term "metastasis" refers to a state in which a malignant tumor has spread from the organ in which it developed to another distant tissue. Therefore, the composition according to the present invention may prevent and treat the spread of breast cancer throughout the body by inhibiting metastasis of breast cancer.

In the present invention, "tumor" is used in the same meaning as "cancer," and refers to a condition typically characterized by uncontrolled cell growth, migration, and proliferation. The cancer according to the present invention is breast cancer, and includes primary and recurrent cancers, and includes both benign and malignant tumors. In the present invention, the breast cancer means all tumors occurring in the breast, and clinically, breast cancer may be classified into three subgroups according to the presence or absence of expression of major genes: 1) estrogen receptor-positive (ER-positive), which expresses estrogen receptor (ER), 2) HER2-positive, which expresses HER2, and 3) triple-negative breast cancer, which does not express all three genes of estrogen receptor, progesterone receptor (PR), and HER2. Additionally, the breast cancer may be classified into non-invasive cancer and invasive cancer according to whether the basement membrane is invaded, and may be classified into ductal carcinoma, lobular carcinoma, and the like according to the mammary tissue in which it occurs.

In the present invention, the breast cancer may be one or more selected from the group consisting of breast ductal carcinoma in situ, inflammatory breast cancer, triple-negative breast cancer, invasive ductal carcinoma, invasive lobular carcinoma, ductal carcinoma in situ (non-invasive), lobular carcinoma in situ (non-invasive), Paget's disease of the breast, invasive breast cancer, and metastatic breast cancer, and according to one example of the present invention, the breast cancer may be one or more selected from the group consisting of breast ductal carcinoma in situ (SUM149 cells, SUM159 cells, and CAL51 cells), inflammatory breast cancer (BCX010 cells and FC-IBC02 cells), triple-negative breast cancer (BT20 cells, MDA-MB-231-LM3 cells, PyMT-N cells, HCC 1806 cells, and HCC 1937 cells), and metastatic breast cancer (MDA-MB-436 cells), but is not limited thereto.

The "breast carcinoma *in situ*" is a type of non-invasive breast cancer in which cancer cells are confined only within epithelial tissue and do not invade the basement membrane of the duct, and includes lobular carcinoma *in situ* and ductal carcinoma *in situ*.

The "inflammatory breast cancer" is one of the most fatal forms of advanced breast cancer, in which cancer cells directly and extensively invade the breast skin to cause symptoms similar to inflammation of the breast, and is a type of invasive breast cancer.

The "triple-negative breast cancer (TNBC)" is breast cancer in which estrogen and progesterone receptors are deficient (ER-/PR-) and the HER2 gene is not expressed (HER2-), is characterized by having resistance to taxol, tamoxifen, and a HER2 activity inhibitor (trastuzumab), and is known to overexpress EGFR, which is a cancer cell growth factor.

The "metastatic breast cancer" means stage 4 breast cancer in which cancer cells have metastasized to other organs of the human body and complete cure is difficult, and is known to have a 5-year survival rate of only 22%. A treatment method is determined in consideration of the presence or absence of a hormone receptor (HR) or human epidermal growth factor receptor 2 (HER-2), the period of cancer recurrence, the progression rate of metastasis, the metastatic site, and the like.

The "invasive breast cancer" is cancer that invades the basement membrane of a duct or lobule, and includes invasive ductal carcinoma, invasive lobular carcinoma, mucinous carcinoma, medullary carcinoma, tubular carcinoma, adenoid cystic carcinoma, secretory carcinoma, apocrine carcinoma, metaplastic carcinoma, and the like.

Furthermore, the breast cancer according to the present invention may be breast cancer for which amelioration or treatment is expected by simultaneously inhibiting ANO1 and EGFR. Preferably, the breast cancer according to the present invention may be associated with one or more mutations selected from the group consisting of EGFR (epidermal growth factor receptor) and ANO1 (Anoctamin 1). That is, the cancer according to the present invention may be breast cancer having a mutation of EGFR and/or ANO1. The mutation of EGFR and/or ANO1 includes amplification of the gene of EGFR and/or ANO1, overexpression of the protein, hyperactivation of the protein, continuous activation of the protein, and the like. That is, the breast cancer according to the present invention may have higher expression or activity of EGFR and/or ANO1 than normal cells. Alternatively, the breast cancer according to the present invention may be one in which an activated state of EGFR and/or ANO1 is sustained. Since the compound according to the present invention may simultaneously inhibit expression and activity of ANO1 and EGFR, the compound may exhibit a particularly excellent anti-cancer effect on breast cancer accompanied by a mutation of EGFR and/or ANO1.

In the present invention, the compound or a pharmaceutically acceptable salt thereof may simultaneously inhibit EGFR and ANO1, but is not limited thereto.

In the present invention, the compound or a pharmaceutically acceptable salt thereof may satisfy one or more properties selected from the group consisting of the following, but is not limited thereto:
(a) inhibiting proliferation or growth of cancer cells;
(b) inhibiting migration of cancer cells;
(c) inhibiting tumorigenicity of cancer cells;
(d) inhibiting metastasis of cancer; and
(e) inhibiting resistance of cancer to an anti-cancer agent.

In the present invention, in (e), the anti-cancer agent may be a chemical anti-cancer agent and/or a targeted anti-cancer agent, but is not limited thereto.

The present inventors confirmed through specific examples that breast cancer cells treated with the compound according to the present invention exhibited inhibited growth, proliferation, migration, tumorigenicity, and metastasis (invasiveness).

In the present invention, "tumorigenicity" means an ability or tendency to form cancer, and according to one example of the present invention, tumorigenicity was shown to be inhibited in a concentration-dependent manner of the compound in breast cancer cells (SUM149, BCX010, BT20, and MDA-MB-231-LM3) treated with the compound according to the present invention.

Furthermore, the present inventors confirmed that the compound of the present invention may simultaneously inhibit ANO1 and EGFR (that is, dual inhibition), and therefore, the compound of the present invention may exhibit a more excellent anti-cancer effect than a single inhibitor of ANO1 or EGFR, and in particular, may inhibit development of resistance of cancer cells to an anti-cancer agent through inhibition of ANO1 and may enhance the anti-cancer effect of the anti-cancer agent. For example, the present inventors confirmed through experiments that when the compound of the present invention was co-administered with paclitaxel, which is a taxane-based chemical anti-cancer agent, a high synergistic effect was exhibited despite the fact that the breast cancer cells (SUM149 and HCC1806) were resistant to paclitaxel. In addition, the present inventors confirmed that the compound of the present invention inhibits growth and tumorigenicity and the like of breast cancer cells SUM149 resistant to paclitaxel (a taxane-based chemical anti-cancer agent), olaparib (a PARP inhibitor), and sacituzumab govitecan (a TROP-2-targeting antibody), BCX010 and BT20 resistant to paclitaxel, and breast cancer cells CAL51 resistant to doxorubicin.

Therefore, in the present invention, the pharmaceutical composition may be administered in combination with a chemical anti-cancer agent or a targeted anti-cancer agent, and the pharmaceutical composition may be administered simultaneously, separately, or sequentially with the anti-cancer agent, but is not limited thereto.

In the present invention, the term "chemical anti-cancer agent" collectively refers to agents that mainly act directly on DNA to block DNA replication, transcription, and translation processes or interfere with synthesis of nucleic acid precursors in metabolic pathways and inhibit cell division, thereby exhibiting anti-cancer activity, that is, cytotoxicity against cancer cells, and in the present invention, the chemical anti-cancer agent may include paclitaxel and docetaxel as "taxane-based chemical anti-cancer agents" that inhibit proliferation by interfering with cell division of cancer cells by disrupting separation of microtubules, which are cellular organelles involved in division and self-replication during the process of cell division; and dactinomycin, doxorubicin, daunorubicin, mitomycin, and bleomycin as "antibiotic-based chemical anti-cancer agents," which are extracted from Streptomyces species, which are soil fungi, and are cell cycle non-specific drugs that intercalate between base pairs of DNA to unwind the DNA helix and interfere with DNA and RNA synthesis. That is, in the present invention, the chemical anti-cancer agent may be one or more selected from the group consisting of paclitaxel, docetaxel, dactinomycin, doxorubicin, daunorubicin, mitomycin, and bleomycin, but is not limited thereto.

In the present invention, the term "targeted anti-cancer agent" means an agent that exhibits an anti-cancer effect by targeting a protein or gene specifically altered in cancer cells or cancer tissues and interfering with molecular activities involved in growth and development of cancer. The targeted anti-cancer agent may be one or more selected from the group consisting of a targeted anti-cancer agent for EGFR, a targeted anti-cancer agent for TROP-2, and a targeted anti-cancer agent for PARP, and the targeted anti-cancer agent for EGFR (EGFR inhibitor) may be cetuximab, osimertinib, gefitinib, afatinib, erlotinib, or lazertinib; the targeted anti-cancer agent for PARP (PARP inhibitor) may be olaparib, niraparib, rucaparib, or talazoparib; and the targeted anti-cancer agent for TROP-2 may be sacituzumab govitecan, but is not limited thereto, and any substance targeting EGFR, PARP, or TROP-2 may be included without limitation.

Additionally, the compound according to the present invention may enhance the anti-cancer effect of an anti-cancer agent by increasing activity of the anti-cancer agent against breast cancer or increasing responsiveness of cancer cells to the anti-cancer agent.

The enhanced responsiveness of cancer cells to the anti-cancer agent means that inhibition of growth, inhibition of proliferation, inhibition of migration, inhibition of tumorigenicity, and inhibition of metastasis of cancer cells by the anti-cancer agent are further enhanced.

In the present invention, "enhancing an anti-cancer effect" refers to all effects that may consequently strengthen the function of an anti-cancer agent, and is a concept including not only enhancing the anti-cancer effect of the anti-cancer agent, such as inhibition of growth of cancer cells, inhibition of metastasis of cancer, and inhibition of recurrence of cancer, but also consequently enhancing the anti-cancer effect by inhibiting resistance or formation of resistance of cancer cells to the anti-cancer agent.

Therefore, the compound according to the present invention may be used as a compound for co-administration with a known anti-cancer agent for the purpose of enhancing the anti-cancer effect of the anti-cancer agent.

For example, the present invention provides a pharmaceutical composition for prevention or treatment of breast cancer, comprising, as active ingredients, (i) the compound represented by Chemical Formula 1 or a pharmaceutically acceptable salt thereof; and (ii) one or more anti-cancer agents selected from the group consisting of chemical anti-cancer agents and targeted anti-cancer agents.

In the present invention, the compound or a pharmaceutically acceptable salt thereof may inhibit resistance of cancer cells to the anti-cancer agent, but is not limited thereto.

In the present invention, the composition may be in the form of a mixture in which the compound or a pharmaceutically acceptable salt thereof; and the anti-cancer agent are mixed, and may be in a form for simultaneous administration of the compound or a pharmaceutically acceptable salt thereof; and the anti-cancer agent.

In the present invention, the composition may be in a form in which the compound or a pharmaceutically acceptable salt thereof; and the anti-cancer agent are each formulated to be administered simultaneously, separately, or sequentially. In this case, the composition may be a pharmaceutical composition for combined administration for simultaneous or sequential administration, comprising a first pharmaceutical composition comprising, as an active ingredient, the compound or a salt thereof in a pharmaceutically effective amount; and a second pharmaceutical composition comprising, as an active ingredient, the anti-cancer agent in a pharmaceutically effective amount. At this time, in the case of sequential administration, the order of administration is not limited, and the dosage regimen may be appropriately adjusted according to the condition of the patient and the like. That is, when the pharmaceutical composition is a pharmaceutical composition for combined administration for sequential administration, the composition may be one in which the compound or a salt thereof ("first component") is administered first and then the anti-cancer agent ("second component") is administered, and the reverse order is also possible.

Additionally, since the compound of the present invention may enhance the anti-cancer effect of an anti-cancer agent by increasing activity of the anti-cancer agent against breast cancer or increasing responsiveness of cancer cells to the anti-cancer agent, the present invention may provide a pharmaceutical composition for enhancing an anti-cancer effect of an anti-cancer agent on breast cancer, comprising, as an active ingredient, the compound represented by Chemical Formula 1 or a pharmaceutically acceptable salt thereof.

In the present invention, the anti-cancer agent may be one or more selected from the group consisting of chemical anti-cancer agents and targeted anti-cancer agents, but is not limited thereto.

In the present invention, the chemical anti-cancer agent may be one or more selected from the group consisting of paclitaxel, docetaxel, dactinomycin, doxorubicin, daunorubicin, mitomycin, and bleomycin, but is not limited thereto. According to one example of the present invention, the chemical anti-cancer agent may be paclitaxel, but is not limited thereto.

In the present invention, the targeted anti-cancer agent may be one or more selected from the group consisting of a targeted anti-cancer agent for EGFR, a targeted anti-cancer agent for TROP-2, and a targeted anti-cancer agent for PARP, but is not limited thereto.

In the present invention, the pharmaceutical composition may be administered simultaneously, separately, or sequentially with the anti-cancer agent, and the order of administration is not limited, and the dosage regimen may be appropriately adjusted according to the type of cancer, the type of anti-cancer agent, the condition of the patient, and the like.

The content of the compound in the composition of the present invention may be appropriately adjusted according to the symptoms of the disease, the degree of progression of the symptoms, the condition of the patient, and the like, and may be, for example, 0.0001 to 99.9 wt% or 0.001 to 50 wt% based on the total weight of the composition, but is not limited thereto. The content ratio is a value based on the dry weight from which the solvent has been removed.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO3) carbon dioxide gas, sodium metabisulfite (Na2S2O5), sodium sulfite (Na2SO3), nitrogen gas (N2), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the age, sex, and body weight of a patient, and generally, 0.001 to 150 mg per 1 kg of body weight, preferably 0.01 to 100 mg, may be administered daily or every other day, or may be administered one to three times per day. However, the dosage may be increased or decreased depending on the administration route, severity of the disease, sex, body weight, age, and the like, and therefore, the above dosage does not limit the scope of the present invention in any way.

As used herein, the "subject" refers to a subject in need of treatment of a disease, and more specifically, refers to a mammal such as a human or a non-human primate, a mouse, a rat, a dog, a cat, a horse, and a cow.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

Additionally, the present invention provides a kit for prevention or treatment of breast cancer, comprising the pharmaceutical composition according to the present invention and an instruction.

The kit according to the present invention may further comprise, without limitation, other components, compositions, solutions, devices, and the like that are ordinarily required for prevention or treatment of breast cancer, in addition to the compound and the anti-cancer agent, and in particular, may comprise an instruction indicating proper use and storage of the compound according to the present invention and the like. All components comprised in the kit may be used one or more times without limitation on the number of times, there is no limitation on the order of using each material, and application of each material may be performed simultaneously or at different times.

Additionally, the kit of the present invention may further comprise a container in addition to the compound and the anti-cancer agent. The container may serve to package the configuration, and may also serve to store and secure the configuration. The material of the container may take the form of, for example, a bottle, tub, sachet, envelope, tube, ampoule, or the like, and these may be formed partially or entirely from plastic, glass, paper, foil, wax, and the like. The container may be fitted with a completely or partially removable closure that is initially a part of the container or may be attached to the container by mechanical, adhesive, or other means, and may also be fitted with a stopper through which the contents may be accessed by a hypodermic needle. The kit may comprise an outer package, and the outer package may comprise instructions regarding use of the components, but is not limited thereto.

In the present specification, "active ingredient" means a component that exhibits intended activity by itself or that may exhibit intended activity together with a carrier and the like that itself has no activity.

Terms or words used in the present specification and claims should not be construed as being limited to ordinary or dictionary meanings, and should be construed as meanings and concepts consistent with the technical spirit of the present invention based on the principle that the inventor may appropriately define the concept of a term in order to describe the inventor's own invention in the best manner.

### [Modes of the Invention]

Hereinafter, preferred examples are presented to help understand the present invention. However, the following examples are provided only to help understand the present invention more easily, and the contents of the present invention are not limited by the following examples.

### [Examples]

### Example 1. Synthesis of Compound AON-MG23 of the present invention

### <Step 1> Synthesis of N-cyclopropyl-N-(2-nitrophenyl)methanesulfonamide

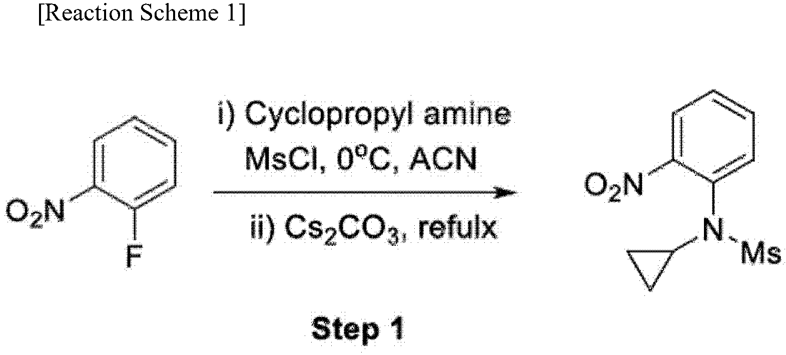

1-Fluoro-2-nitrobenzene (1.00 eq.) and cyclopropylamine (1.00 eq.) were dissolved in acetonitrile (ACN), methanesulfonyl chloride (MsCl, 1.0 eq.) was slowly added at 0 °C, and the resulting solution was stirring at the same temperature for one hour. Thereafter, cesium carbonate (Cs₂CO₃, 5.00 eq.) was added at the same temperature, and then the resulting mixture was stirred under reflux overnight. After the reaction was complete, the temperature was lowered to room temperature, and extraction was performed using ethyl acetate and water. Anhydrous sodium sulfate was added to the organic layer and stirred, the resulting mixture was filtered using a filter, and the filtrate was concentrated under reduced pressure. The concentrated residue was purified by column chromatography (hexane:ethyl acetate = 3:1) to synthesize N-cyclopropyl-N-(2-nitrophenyl)methanesulfonamide as a pale yellow solid, and the yield was 36%.

### <Step 2> Synthesis of N-(2-aminophenyl)-N-cyclopropylmethanesulfonamide

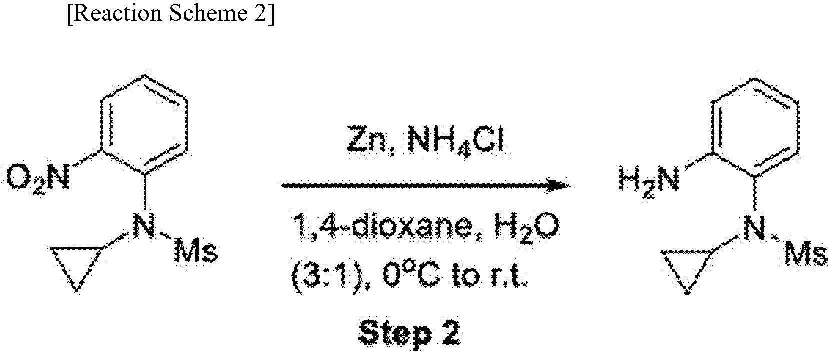

N-cyclopropyl-N-(2-nitrophenyl)methanesulfonamide (1.00 eq.) synthesized in Step 1 was added to 1,4-dioxane and water (3:1), and the resulting mixture was stirred. Thereafter, the reactor was cooled to 0 °C, and zinc (Zn, 10.0 eq.) and ammonium chloride (NH₄Cl, 10.0 eq.) were added. Thereafter, the temperature was gradually increased while stirring for four hours. After the reaction was complete, the mixture was filtered using Celite, and the filtrate was extracted with ethyl acetate and water. Anhydrous sodium sulfate was added to the organic layer and stirred, the resulting mixture was filtered using a filter, and the filtrate was concentrated under reduced pressure. The concentrated residue was used in Step 3 without a separate purification process.

### <Step 3> Synthesis of N-cyclopropyl-N-(2-((2,5-dichloropyrimidine-4-yl)amino)phenyl)methanesulfonamide

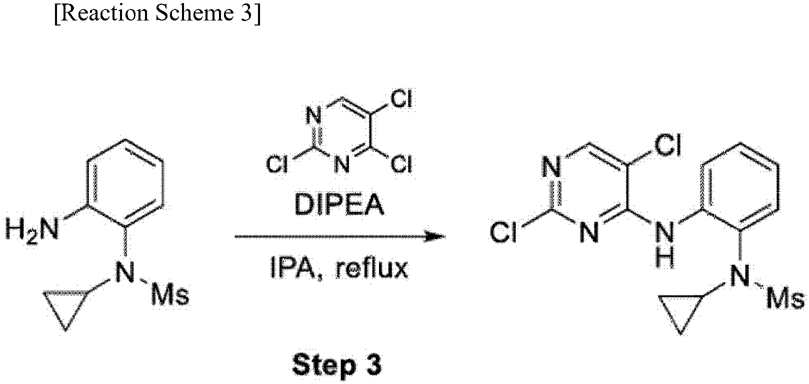

N-(2-aminophenyl)-N-cyclopropylmethanesulfonamide (1.00 eq.) synthesized in Step 2 was added to isopropyl alcohol (IPA), and 2,4,5-trichloropyrimidine (1.1 eq.) and N,N-diisopropylethylamine (DIPEA, 2.5 eq.) were added at room temperature. Thereafter, the resulting mixture was stirred overnight under reflux. After the reaction was complete, the mixture was evaporated under reduced pressure and extracted using water and dichloromethane. The organic layer was washed with 2 N hydrochloric acid (HCl), anhydrous sodium sulfate was added to the organic layer, and the resulting mixture was stirred. Thereafter, the mixture was filtered using a filter, and the filtrate was concentrated under reduced pressure. The concentrated residue was purified by column chromatography (hexane:ethyl acetate = 3:1) to synthesize N-cyclopropyl-N-(2-((2,5-dichloropyrimidin-4-yl)amino)phenyl)methanesulfonamide as a pale yellow solid, and the yield was 58.9%.

### <Step 4> Synthesis of N-(2-((5-chloro-2-((4-(4-(dimethylamino)piperidine-1-yl)-2-methoxyphenyl)amino)pyrimidine-4-yl)amino)phenyl)-N-cyclopropylmethanesulfonamide

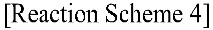

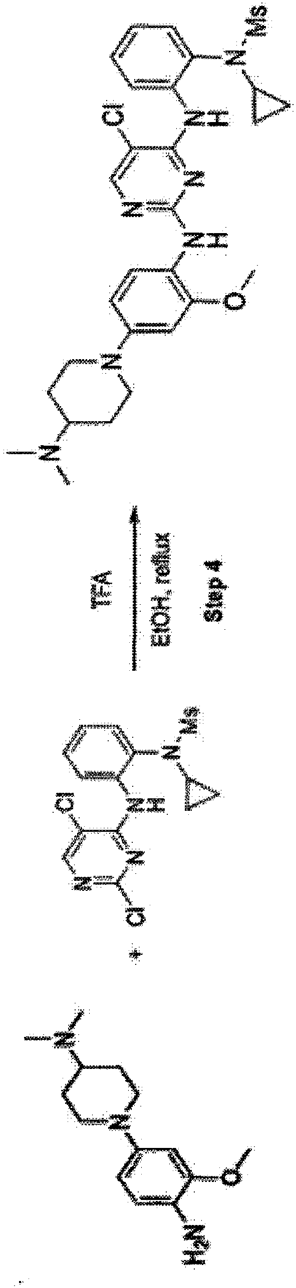

N-cyclopropyl-N-(2-((2,5-dichloropyrimidine-4-yl)amino)phenyl)methanesulfonamide (1.00 eq.) synthesized in Step 3 was added to ethanol (EtOH), 1-(4-amino-3-methoxyphenyl)-N,N-dimethylpiperidin-4-amine (1.00 eq.) and trifluoroacetic acid (TFA, 1.95 eq.) were added at room temperature, and the resulting mixture was stirred overnight under reflux. After the reaction was complete, the mixture was neutralized with a 1 N sodium hydroxide (NaOH) solution and extracted with water and ethyl acetate. Anhydrous sodium sulfate was added to the organic layer, the resulting mixture was mixed, stirred, and then filtered using a filter, and the filtrate was concentrated under reduced pressure. The concentrated residue was purified by column chromatography (hexane:ethyl acetate = 3:1) to synthesize N-(2-((5-chloro-2-((4-(4-(dimethylamino)piperidin-1-yl)-2-methoxyphenyl)amino)pyrimidin-4-yl)amino)phenyl)-N-cyclopropylmethanesulfonamide as a yellow solid (AON-MG23), and the yield was 25%.

¹H nuclear magnetic resonance (NMR) (500 MHz, dimethyl sulfoxide (DMSO)-d6) δ 8.33 (s, 1H), 8.19 (s, 1H), 8.09 (s, 1H), 7.99 (s, 1H), 7.62 (dd, J = 7.8, 1.6 Hz, 1H), 7.35 (d, J = 8.7 Hz, 1H), 7.22 (d, J = 8.1 Hz, 1H), 7.15 (td, J = 7.6, 1.6 Hz, 1H), 6.63 (d, J = 2.5 Hz, 1H), 6.48 (dd, J = 8.7, 2.5 Hz, 1H), 3.75 (s, 3H), 3.72 (d, J = 12.2 Hz, 2H), 3.23 (m, 4H), 2.68 (td, J = 12.1, 2.5 Hz, 2H), 2.22 (s, 6H), 1.86 (d, J = 12.4 Hz, 2H), 1.51 (qd, J = 12.0, 3.9 Hz, 2H), 1.04 - 0.92 (m, 2H), 0.55 - 0.49 (m, 1H), 0.17 - 0.14 (m, 1H). High-resolution mass spectrometry (HRMS): 585.2294, cal: 585.2289.

### Example 2. Confirmation of proliferation inhibitory effect of compound AON-MG23 of the present invention on breast cancer cells

Human breast cancer cell line MDA-MB-231 cells were plated in a 96-well plate at a density of 1 x 10⁵ cells/well and cultured for 24 hours. Thereafter, the cells were treated with compound AON-MG23 of the present invention at various concentrations and cultured for 48 hours. The cells were treated with the compound of the present invention at various concentrations of 0 µM, 0.1 µM, 0.25 µM, 0.5 µM, 1 µM, 2 µM, 4 µM, 8 µM, and 16 µM. 10 µL of cell counting kit (CCK) reagent was added and allowed to react for one hour, and then the absorbance was measured at 450 nm.

As a result, as shown in FIG. 1, it was confirmed that compound AON-MG23 of the present invention inhibited the proliferation of breast cancer cells.

### Example 3. Confirmation of proliferation inhibitory effect of compound AON-MG23 of the present invention on breast cancer cells

To confirm the proliferation inhibitory effect of the compound of the present invention on breast cancer cells, a colony formation test was conducted. Specifically, six breast cancer cell lines (SUM149, BCX010, BT20, MDA-MB-231-LM3, CAL51, PyMT-N) were plated in a 6-well cell culture plate at a density of 250 or 1,000 cells per well. On the next day, the cell culture medium was removed and replaced with a cell culture medium containing compound AON-MG23 of the present invention at concentrations of 0 µM, 0.25 µM, 0.5 µM, and 1 µM. After culturing the cells for 11 to 14 days, the resulting cell colonies were stained and photographed, and then the cell colonies were counted and analyzed using Image J software.

As a result, as shown in FIGS. 2A to 2C, it was confirmed that cell colony formation was inhibited in all six breast cancer cell lines after treatment with compound AON-MG23 of the present invention.

### Example 4. Confirmation of tumorigenicity inhibitory effect of compound AON-MG23 of the present invention on breast cancer cells

To confirm the tumorigenicity inhibitory effect of the compound of the present invention on breast cancer cells, an anchorage-dependent soft agar assay was performed. Specifically, 0.5% agar or 0.7% agar was added to cell culture media diluted with the compound of the present invention at concentrations of 0 nM, 100 nM, 500 nM, and 1000 nM. 2 ml of 0.5% agar containing the compound of the present invention at various concentrations was added to a 6-well cell culture plate and solidified at room temperature. Thereafter, 1 ml of 1 x 10⁴ cells of four breast cancer cell lines (SUM149, BCX010, BT20, MDA-MB-231-LM3) and 1 ml of 0.7% agar containing the compound of the present invention at various concentrations were mixed in a 1:1 ratio to prepare a 0.35% solution, and 2 ml of this solution was placed on top of the solidified 0.5% agar. The mixture was then solidified at room temperature until the upper layer was fixed, and then cells were cultured in an incubator at 37 °C and 5% CO₂ for two weeks. The formed cell clones were stained and photographed, and then the cell clones were counted and analyzed using Image J software.

As a result, as shown in FIGS. 3A and 3B, it was confirmed that the tumorigenicity of the four breast cancer cell lines was inhibited starting from 100 nM of compound AON-MG23 of the present invention.

### Example 5. Confirmation of inhibitory effect of compound AON-MG23 of the present invention on cell migration in breast cancer cells

Human breast cancer cell line MDA-MB-231 cells were seeded in SPLScar Block plates (SPL LIFE SCIENCES) at a density of 7 x 10⁴/well and cultured for 24 hours. After 20 hours, the blocks were removed, and at the same time, compound AON-MG23 of the present invention was applied at various concentrations. Thereafter, cells were cultured in a CO₂ incubator at 37 °C for 24 hours. The cells were treated with the compound of the present invention at various concentrations of 0 µM, 0.5 µM, 1 µM, 2 µM, and 4 µM. Photographs were taken before and after each culture, and the migration distance of the cells was measured using Image J software. Based on the results, the inhibition of the cell migration was analyzed.

As a result, as shown in FIG. 4, it was confirmed that when cells were treated with the compound of the present invention at a concentration of 0.5 µM, the migration of MDA-MB-231 cells was inhibited by 50% compared to the control group.

### Example 6. Confirmation of inhibitory effect of compound AON-MG23 of the present invention on cell migration in breast cancer cells

Breast cancer cell line MDA-MG231-LM3 cells pre-treated with 0.5 µM of compound AON-MG23 of the present invention and cells that were not treated were prepared. As shown in FIG. 5, 6 x 10⁴ cells of both the untreated and pre-treated groups were seeded on top of a membrane filter (Transwell membrane insert, Corning) in a cell culture medium that did not contain 5% fetal bovine serum (FBS). The membrane filter seeded with the cells was placed into a 24-well plate supplemented with a cell culture medium containing fetal bovine serum and cultured for eight hours in an incubator at 37°C and 5% CO₂. Cells that migrated to the bottom surface of the membrane filter were stained and photographed under a microscope, and counted and analyzed using Image J software.

As a result, as shown in FIG. 6, it was confirmed that cell migration was inhibited in the group pre-treated with compound AON-MG23 of the present invention compared to the untreated group.

### Example 7. Confirmation of inhibitory effect of compound AON-MG23 of the present invention on breast cancer cell metastasis

Human breast cancer cell line MDA MB-231 cells were seeded at a density of 6 x 10⁴ cells/well on top of a filter coated with Matrigel (Transwell invasion chamber, Corning), treated with compound AON-MG23 of the present invention (0, 0.5, 1, 2, 4 µM), and cultured in a CO₂ in an incubator at 37 °C for 20 hours. After culture, cell fixation and staining were performed using the Diff-Quick stain Kit (Sysmex, Kobe, Japan). The stained cells were imaged under a microscope, and the cells were counted and statistically analyzed using Image J software.

As a result, as shown in FIG. 7, it was confirmed that the group treated with compound AON-MG23 of the present invention exhibited a reduced degree of penetration into Matrigel compared to the untreated control group, thereby inhibiting the metastatic potential of the cancer cells.

### Example 8. Confirmation of inhibitory effect of compound AON-MG23 of the present invention on breast cancer cell metastasis

Breast cancer cell line MDA-MB-231 was seeded at a density of 6 x 10⁴ cells/well on top of a membrane filter coated with Matrigel(Transwell membrane insert, Corning). Thereafter, the cells were treated with compound AON-MG23 of the present invention at concentrations of 0 µM (DMSO), 0.5 µM, and 1 µM, and cultured in a CO₂ incubator at 37 °C for 24 hours. After 24 hours of culture, the cells on the bottom surface of the membrane filter were stained and photographed under a microscope, and counted and analyzed using Image J software.

As a result, as shown in FIG. 8, it was confirmed that the experimental group treated with compound AON-MG23 of the present invention exhibited a reduced degree of penetration into Matrigel compared to the untreated control group, and the reduction in the degree of Matrigel penetration was dependent on the AON-MG23 treatment concentration.

### Example 9. Confirmation of the effect of compound AON-MG23 of the present invention on the expression of anoctamin-1 (ANO1) and epidermal growth factor receptor (EGFR) proteins in human breast cancer cell lines

Human breast cancer cell line MDA-MB-231 was seeded in 60 mm dishes at a density of 5 × 10⁵ cells/dish and cultured for 24 hours. Thereafter, the cells were treated with compound AON-MG23 of the present invention at concentrations of 0.125 µM, 0.25 µM, 0.5 µM, 1 µM, and 2 µM, while an untreated control group was treated with DMSO and cultured for 48 hours. After culture, the cells were recovered, and the recovered cells were lysed to perform Western blot. Antibodies used for Western blot included an anti-ANO1 antibody (Abcam, ab53212), an anti-EGFR antibody (Cell Signaling, #4267S), and an anti-glyceraldehyde 3-phosphate dehydrogenase (GAPDH) antibody (Cell Signaling, #5174), and protein concentration was quantified using the bovine serum albumin (BSA) method (Protein Quantitation Assay, BSA assay) (Pierce, Cat.23225). After lysing the cells, the proteins obtained therefrom were separated by molecular weight using sodium dodecyl sulfate-polyacrylamide gel electrophoresis (SDS-PAGE) with equal amounts (20 µg). After transferring the proteins to a polyvinylidene fluoride (PVDF) membrane (Bio-Rad), the proteins were treated with a 5% blocking buffer (5% skim milk in Tris-buffered saline buffer containing 0.1% Tween 20 (TBS-T)) at room temperature for one hour. Subsequently, the membrane was treated with a primary antibody, which was allowed to react at 4 °C for 18 hours. After the reaction was complete, the membrane was washed three times with TBS-T for 10 minutes each at room temperature. Subsequently, the membrane was treated with a secondary antibody labeled with horseradish peroxidase at room temperature for one hour and then washed three times with TBS-T for 10 minutes each. After washing, the membrane was treated with an enhanced chemiluminescence (ECL) kit (Thermo, West Pico Plus) to induce a reaction, and the results were visualized using a Da Vinci-Q (Youngin Labplus CO., LTD.) instrument. The visualized results were analyzed by quantifying the amount of each protein using Image J software.

As a result, as shown in FIG. 9, it was confirmed that compound AON-MG23 of the present invention reduced both the ANO1 protein and the EGFR protein in breast cancer cell lines, and protein reduction started from the 1 µM treatment group for EGFR protein and from the 0.5 µM treatment group for ANO1 protein.

### Example 10. Confirmation of drug combination effect of compound AON-MG23 of the present invention on human breast cancer cells

The combination effect of compound AON-MG23 of the present invention was confirmed using Bliss scoring based on the results of the sulforhodamine B assay (SRB assay). Specifically, human breast cancer cell lines SUM149 and HCC1806 were plated in a 96-well cell culture plate at a density of 1 x 10³ cells/well. On the next day, paclitaxel, the drug to be tested for the combination effect with compound AON-MG23 of the present invention, was diluted to concentrations for testing, and cells were cultured for 24 hours. After carefully removing the cell culture medium, the cells were co-treated with AON-MG23 and paclitaxel at different concentrations and cultured for five days. After five days of co-treatment, the culture medium mixed with the drug was removed, and 10% trichloroacetic acid (TCA) was added to each well for fixation at 4 °C for one hour. After removing the TCA, the 96-well plate was washed five times with running water and dried at room temperature for one hour. Thereafter, a 0.4% SRB solution (sulforhodamine B in 1% acetic acid) was added to each well to stain the cells at room temperature for 30 minutes. After removing the 0.4% SRB solution, the plate was washed five times with 1% acetic acid and dried again for one hour. After drying, 200 µL of 10 mM Tris-base (pH 10.5) was added to each well, and the dye that stained the cells was dissolved by stirring at room temperature for 10 minutes. The absorbance of the dissolved solution was measured at 540 nM using a plate reader. The measured absorbance values were compared to the control group, converted into percentages, and saved in an Excel file format. Thereafter, the Excel file including the percentage data was uploaded to https://synergyfinder.fimm.fi/ to analyze the degree of synergistic effect during the combined treatment of the drugs. The presence of a synergistic effect was evaluated according to the following criteria based on relevant literature (Bliss, C. I. (1939). The toxicity of poisons applied jointly. Annals of Applied Biology, 26(3), 585-615.): a score > 0 indicates a synergistic effect; a score = 0 indicates an independent effect; and a score < 0 indicates an antagonistic effect.

As shown in FIGS. 10A and 10B, the results of the Bliss synergy scoring of compound AON-MG23 of the present invention and paclitaxel in human breast cancer cell lines SUM149 and HCC1806 confirmed that a high synergistic effect was observed with 18.743 points in the SUM149 cell line and 9.249 points in the HCC1806 cell line.

The foregoing description of the present invention is intended for illustrative purposes, and it will be understood by those skilled in the art that various modifications can be made thereto in other specific forms without departing from the technical spirit or essential features of the present invention. Therefore, the embodiments described above should be understood as illustrative in all respects and not restrictive.

### [Industrial Applicability]

The novel compound of the present invention not only significantly inhibits the growth, proliferation, migration, tumorigenicity, and metastasis of breast cancer cells, but also may simultaneously inhibit ANO1 and EGFR in breast cancer cells, thereby exhibiting a stronger anti-cancer effect through dual inhibition of the two proteins. Additionally, when used in combination with an anti-cancer agent and the like, the compound may further enhance the efficacy of the anti-cancer agent and may inhibit resistance of cancer. Therefore, the compound itself may be used as a dual-target anti-cancer agent against ANO1 and EGFR, and may also be utilized as a combined preparation with an anti-cancer agent, and thus may be variously utilized in the field of prevention and treatment of breast cancer, and therefore has industrial applicability.

## Claims

1. A pharmaceutical composition for preventing or treating breast cancer, comprising, as an active ingredient, a compound represented by Chemical Formula 1 or a pharmaceutically acceptable salt thereof:

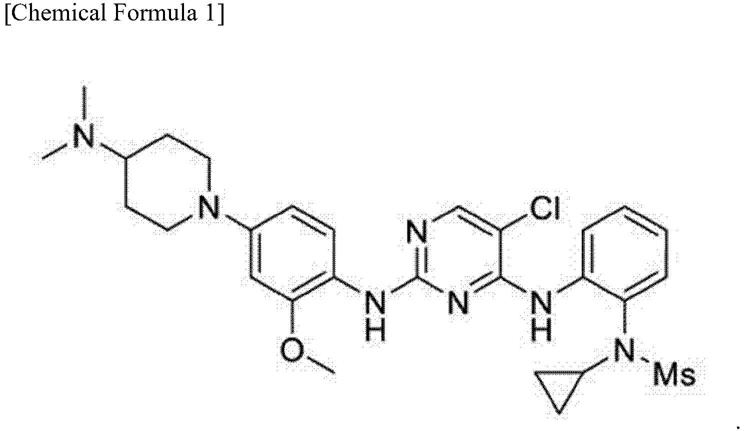

2. The pharmaceutical composition of claim 1, wherein the compound or a pharmaceutically acceptable salt thereof simultaneously inhibits epidermal growth factor receptor (EGFR) and anoctamin-1 (ANO1).

3. The pharmaceutical composition of claim 1, wherein the compound or a pharmaceutically acceptable salt thereof satisfies one or more properties selected from the group consisting of:
(a) inhibiting the proliferation or growth of cancer cells;
(b) inhibiting the migration of cancer cells;
(c) inhibiting the tumorigenicity of cancer cells;
(d) inhibiting the metastasis of cancer; and
(e) inhibiting the resistance of cancer to anti-cancer agents.

4. The pharmaceutical composition of claim 3, wherein the anti-cancer agent is a chemical anti-cancer agent or a targeted anti-cancer agent.

5. The pharmaceutical composition of claim 4, wherein the chemical anti-cancer agent is one or more selected from the group consisting of paclitaxel, docetaxel, dactinomycin, doxorubicin, daunorubicin, mitomycin, and bleomycin.

6. The pharmaceutical composition of claim 4, wherein the targeted anti-cancer agent is one or more selected from the group consisting of a targeted anti-cancer agent for EGFR, a targeted anti-cancer drug for trophoblast cell-surface antigen 2 (TROP-2), and a targeted anti-cancer agent for poly(ADP-ribose) polymerase (PARP).

7. The pharmaceutical composition of claim 1, wherein the breast cancer is one or more selected from the group consisting of breast ductal carcinoma *in situ*, inflammatory breast cancer, triple-negative breast cancer, invasive ductal carcinoma, invasive lobular carcinoma, ductal carcinoma *in situ* (non-invasive), lobular carcinoma *in situ* (non-invasive), Paget's disease of the breast, invasive breast cancer, and metastatic breast cancer.

8. The pharmaceutical composition of claim 1, wherein the pharmaceutical composition is administered in combination with a chemical anti-cancer agent or a targeted anti-cancer agent.

9. The pharmaceutical composition of claim 8, wherein the pharmaceutical composition is administered simultaneously, separately, or sequentially with the anti-cancer agent.

10. A pharmaceutical composition for preventing or treating breast cancer, comprising as an active ingredient:
(i) a compound represented by Chemical Formula 1 or a pharmaceutically acceptable salt thereof; and
(ii) one or more anti-cancer agents selected from the group consisting of chemical anti-cancer agents and targeted anti-cancer agents:

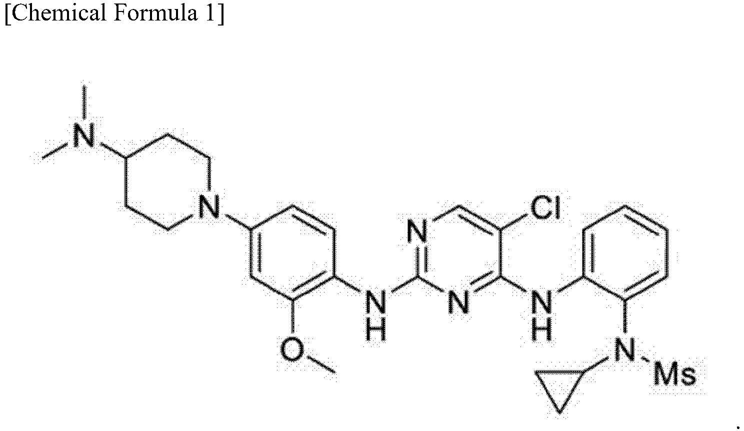

11. The pharmaceutical composition of claim 10, wherein the compound or a pharmaceutically acceptable salt thereof inhibits the resistance of cancer cells to the anti-cancer agent.

12. The pharmaceutical composition of claim 10, wherein the composition is in the form of a mixture mixing the compound or a pharmaceutically acceptable salt thereof; and the anti-cancer agent.

13. The pharmaceutical composition of claim 10, wherein the composition is in a form in which the compound or a pharmaceutically acceptable salt thereof; and the anti-cancer agent are each formulated to be administered simultaneously, separately, or sequentially.

14. A pharmaceutical composition for enhancing an anti-cancer effect of an anti-cancer agent on breast cancer, comprising, as an active ingredient, a compound represented by Chemical Formula 1 or a pharmaceutically acceptable salt thereof:

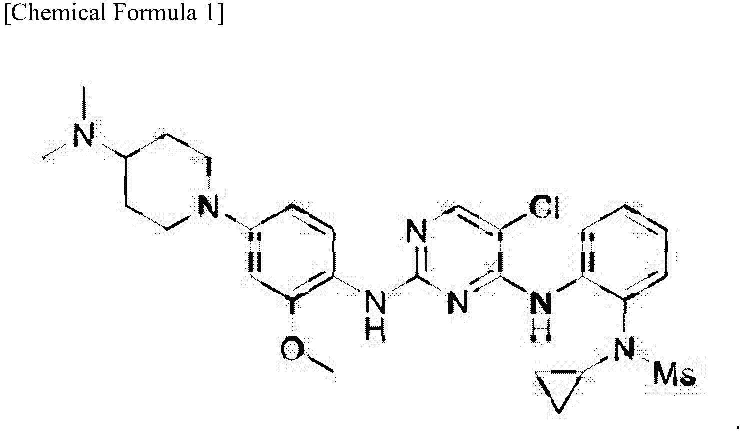

15. The pharmaceutical composition of claim 14, wherein the anti-cancer agent is one or more selected from the group consisting of a chemical anti-cancer agent and a targeted anti-cancer agent.

16. The pharmaceutical composition of claim 15, wherein the chemical anti-cancer agent is one or more selected from the group consisting of paclitaxel, docetaxel, dactinomycin, doxorubicin, daunorubicin, mitomycin, and bleomycin.

17. The pharmaceutical composition of claim 15, wherein the targeted anti-cancer agent is one or more selected from the group consisting of a targeted anti-cancer agent for EGFR, a targeted anti-cancer drug for trophoblast cell-surface antigen 2 (TROP-2), and a targeted anti-cancer agent for poly(ADP-ribose) polymerase (PARP).

18. The pharmaceutical composition of claim 14, wherein the pharmaceutical composition is administered simultaneously, separately, or sequentially with the anti-cancer agent.

19. A method of preventing, ameliorating, or treating breast cancer, comprising a step of administering a compound represented by Chemical Formula 1 or a pharmaceutically acceptable salt thereof; or a composition comprising the same, in a pharmaceutically effective amount to a subject in need thereof:

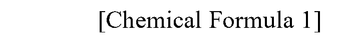

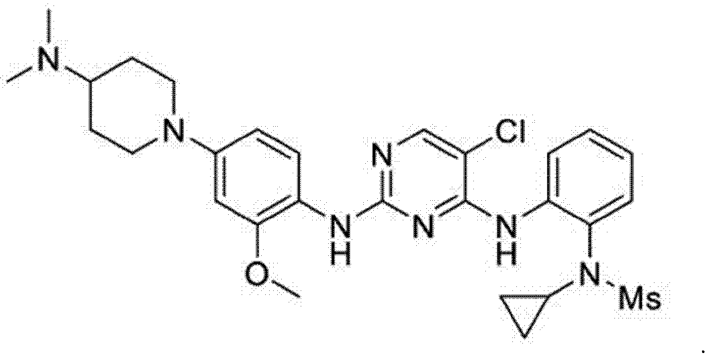

20. A use of a compound represented by Chemical Formula 1 or a pharmaceutically acceptable salt thereof; or a composition comprising the same for preventing, ameliorating, or treating breast cancer:

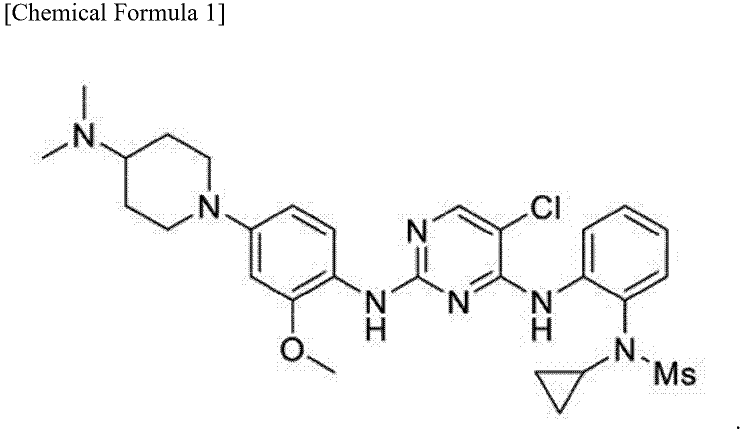

21. A use of a compound represented by Chemical Formula 1 or a pharmaceutically acceptable salt thereof; or a composition comprising the same for preparing a preparation for preventing, ameliorating, or treating breast cancer:

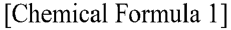

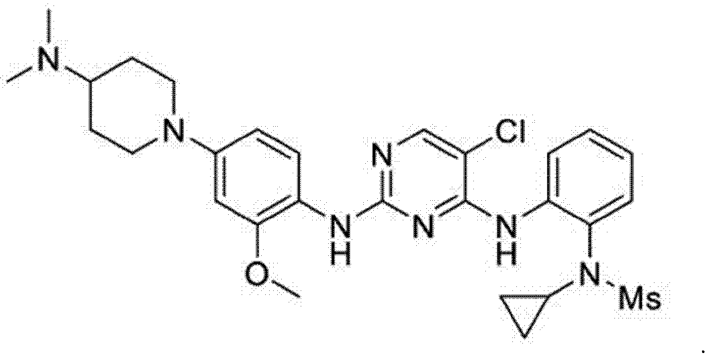

22. A method of enhancing an anti-cancer effect of an anti-cancer agent on breast cancer, comprising a step of administering a compound represented by Chemical Formula 1 or a pharmaceutically acceptable salt thereof; or a composition comprising the same, in a pharmaceutically effective amount to a subject in need thereof:

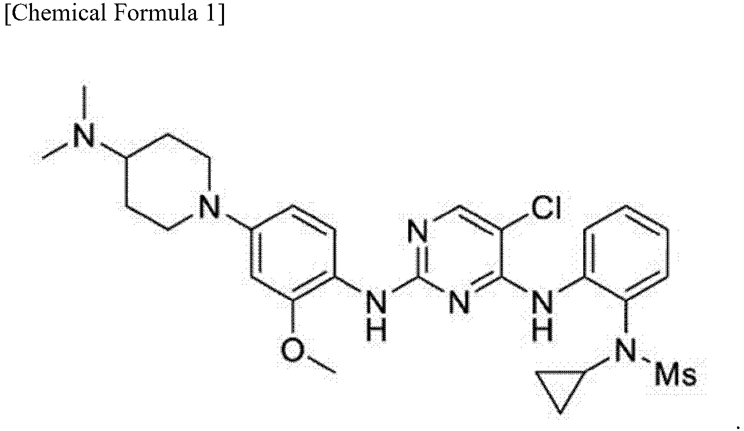

23. A use of a compound represented by Chemical Formula 1 or a pharmaceutically acceptable salt thereof; or a composition comprising the same for enhancing an anti-cancer effect of an anti-cancer agent on breast cancer:

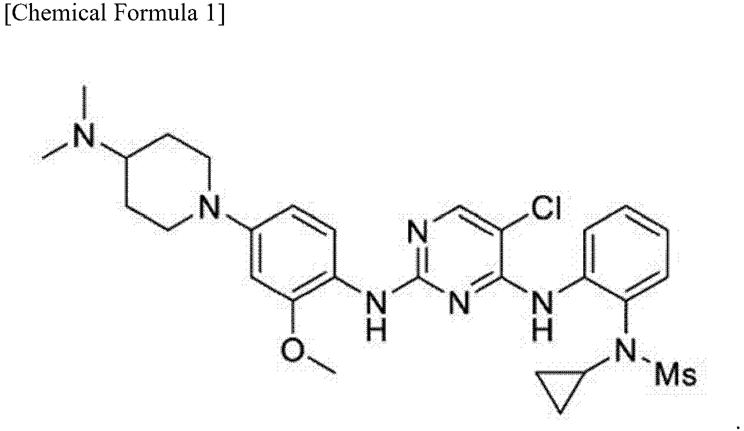

24. A use of a compound represented by Chemical Formula 1 or a pharmaceutically acceptable salt thereof; or a composition comprising the same for preparing a preparation for enhancing an anti-cancer effect of an anti-cancer agent on breast cancer:

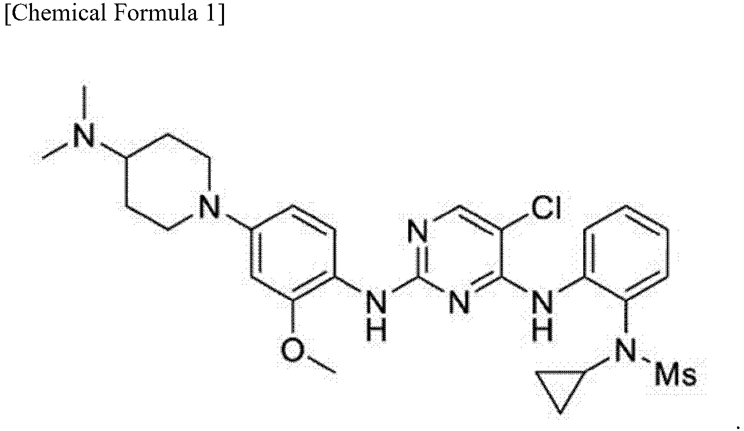

25. A method of preventing, ameliorating, or treating breast cancer, comprising a step of administering (i) a compound represented by Chemical Formula 1 or a pharmaceutically acceptable salt thereof; and (ii) one or more anti-cancer agents selected from the group consisting of chemical anti-cancer agents and targeted anti-cancer agents; or a composition comprising the same, in a pharmaceutically effective amount to a subject in need thereof:

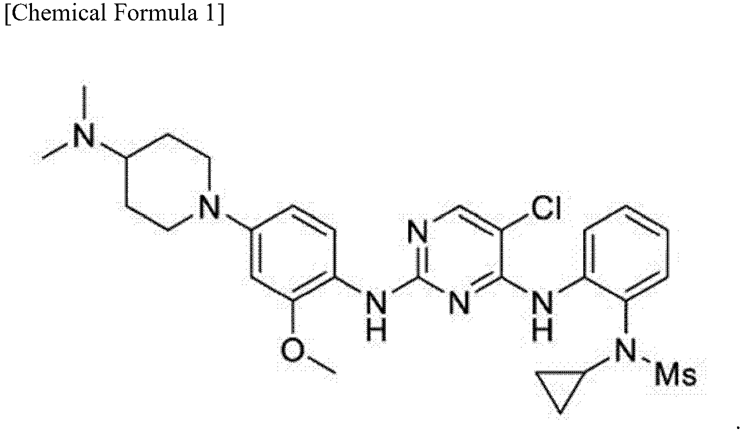

26. A use of (i) a compound represented by Chemical Formula 1 or a pharmaceutically acceptable salt thereof; and (ii) one or more anti-cancer agents selected from the group consisting of chemical anti-cancer agents and targeted anti-cancer agents; or a composition comprising the same for preventing, ameliorating, or treating breast cancer:

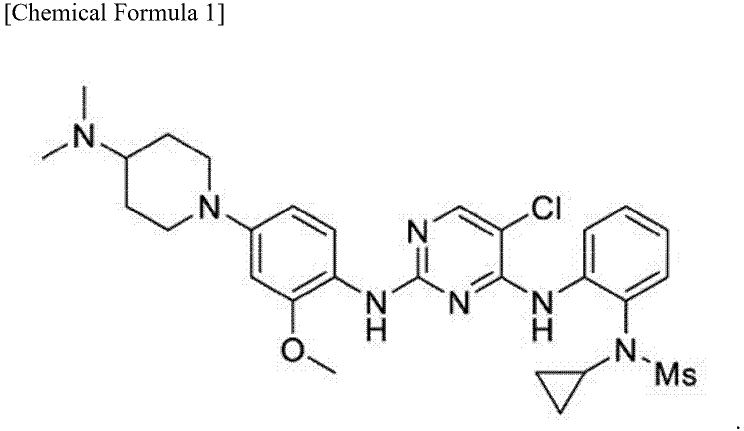

27. A use of (i) a compound represented by Chemical Formula 1 or a pharmaceutically acceptable salt thereof; and (ii) one or more anti-cancer agents selected from the group consisting of chemical anti-cancer agents and targeted anti-cancer agents; or a composition comprising the same for preparing a preparation for preventing, ameliorating, or treating breast cancer:
